# EUROPEAN PATENT SPECIFICATION

(11) **EP 1 443 982 B1**
(45) Date of publication and mention of the grant of the patent: **14.02.2007**
(21) Application number: 02789509.3
(22) Date of filing: 07.11.2002
(51) Int. Cl.: A61L 27/56, A61L 27/14, C08J 9/00

(54) **POROUS POLYMERIC PROSTHESES AND METHODS FOR MAKING SAME**
PORÖSE POLYMERPROSTHESEN UND DEREN HERSTELLUNGSVERFAHREN
PROTHESES POLYMERIQUES POREUSES ET PROCEDES DE FABRICATION

(30) Priority: 08.11.2001 US 10304
(43) Date of publication of application: 11.08.2004
(73) Proprietor: KENSEY NASH CORPORATION, Exton, PA 19341 (US)
(72) Inventor: RINGEISEN, Timothy, A., Exton, PA 19314 (US)
(74) Representative: Andrae, Steffen
(86) International application number: PCT/US2002/035848
(87) International publication number: WO 2003/039615

(56) References cited:
- EP-A- 0 216 149
- EP-A- 0 308 102
- EP-A- 0 382 356
- EP-A- 0 396 809
- WO-A-00/55300
- WO-A-96/37165
- US-A- 3 492 154
- US-A- 3 527 653
- US-A- 3 553 008
- US-A- 4 355 426
- US-A- 4 657 544
- US-A- 5 229 045
- DATABASE WPI Section Ch, Week 197632 Derwent Publications Ltd., London, GB; Class A25, AN 1976-60950X XP002231746 & JP 51 074058 A (MITSUBISHI RAYON CO LTD) , 26 June 1976 (1976-06-26)
- DATABASE WPI Section Ch, Week 200109 Derwent Publications Ltd., London, GB; Class A18, AN 2001-075349 XP002231747 & JP 2000 319434 A (NITTO DENKO CORP), 21 November 2000 (2000-11-21)
- DATABASE WPI Section Ch, Week 197547 Derwent Publications Ltd., London, GB; Class A82, AN 1975-77632W XP002249146 & JP 50 042004 A (KURARAY CO LTD), 16 April 1975 (1975-04-16)
- DATABASE WPI Section Ch, Week 198350 Derwent Publications Ltd., London, GB; Class A18, AN 1983-841300 XP002249147 & JP 58 189242 A (TOYO POLYMER KK), 4 November 1983 (1983-11-04)

## Description

### TECHNICAL FIELD

The present invention relates to an improved porous polymer useful for various applications in industry, including the medical industry, for example, as a biological prosthesis and particularly useful in vascular surgery. The porous polymer can be made by use of a new gel enhanced phase separation technique, which, among other advantages, permits enhanced shape-making capability.

### BACKGROUND ART

The present invention encompassing polymer engineering and processing came about from efforts to improve existing properties of porous polymers, including medical devices and prostheses and, in particular, medical devices (e.g., vascular grafts). Accordingly, a review of the vascular graft art is appropriate.

The search for the ideal blood vessel substitute has to date focused on biological tissues and synthetics. Despite intensive efforts to improve the nature of blood vessel substitutes many problems remain, such as increasing failure rate with decreasing caliber of the blood vessel substitute, a high failure rate when infection occurs, and aneurysm formation. The major need for vascular grafts is for adequate supply of blood to organs and tissues whose blood vessels are inadequate either through defects, trauma or diseases. Vascular grafts are also needed to provide access to the bloodstream for individuals undergoing hemodialysis. The three major types of vascular grafts are peripheral, arterial-to-venous access, and endovascular.

Peripheral grafts are those used in the neck and extremities, with the most common being used in the leg. This results in supply problems being some intermediate and most small diameter arteries are replaced or bypassed using an autologous saphenous vein, the long vein extending down the inside of the leg, with a secondary source being the radial veins of the arms. In a given patient, suitable veins may be absent, diseased or too small to be used, and removal of the vein is an additional surgical procedure that carries attendant risk.

Additionally, arterial-to-venous access grafts are used to access the circulatory system during hemodialysis. Vascular grafts used in connection with hemodialysis are attached to an artery at one end and sewn to a vein at the other. Two large needles are inserted into the graft. One needle removes the blood where it flows through an artificial kidney machine and is then returned to the body via the second needle. Normal kidney function is destroyed by several acute and chronic diseases, including diabetes and hypertension. Patients suffering from kidney failure are maintained by dialysis three times a week for approximately four hours per session. Due to the constant punishment these grafts undergo, there is a high occurrence of thrombosis, bleeding, infections, and pseudoaneurysm

Endovascular grafts are used to reline diseased or damaged arteries, particularly those in which aneurysms have formed, in a less invasive manner than standard vascular surgical procedures. Various surgical techniques and materials have been developed to replace and repair blood vessels. Ideally, the thickness of the prosthesis is minimized, so that it can be delivered to the implantation site using a percutaneous procedure, typically catheterization and kept in place utilizing stents. Problems associated with this type of implantation include thrombosis, infection and new aneurysm formation at the location of the stent.

Initially, autografts were used to restore continuity; however, limited supply and inadequate sizes forced the use of allografts from both donor and umbilical cord harvest such as that described in U.S. Patent 3,974,526. Development of aneurysms and arteriosclerosis as well as the fear of disease transmission necessitated the search for a better substitute.

Artificial vascular grafts are well known in the art. See for example U.S. Patent No. 5,747,128; U.S. Patent No. 5,716,395; U.S. Patent No. 5,700,287; U.S. Patent No. 5,609,624; U.S. Patent No. 5,246,452 and U.S. Patent No. 4,955,899. Development of two different fibrous and pliable synthetic plastic cloths revolutionized vascular reconstructive surgeries. Whenever suitable autograft was not available woven grafts of polyethylene terephthalate (Dacron®) and drawn out polytetrafluoroethylene (Teflon®) fibrils as defined in U.S. Patents 3,953,566; 4,187,390 and 4,482,516 were used. Even though these products were widely used they did have many drawbacks including infection, clot formation, occlusions and the inability to be used in grafts smaller than 6 mm inside diameter due to clotting. Additionally, the graft had to be porous enough so that tissue ingrowth could occur, yet have a tight enough weave to the fibers so that hemorrhage would not occur. This made it necessary to pre-clot these grafts prior to use. Recently, vascular prostheses have been coated with bioabsorbable substances such as collagen, albumin, or gelatin during manufacture instead of preclotting at surgery. For purposes of this patent disclosure, the term "bioabsorbable" will be considered to be substantially equivalent to "bioresorbable", "bioerodable", "absorbable" and "resorbable".

Compliance problems with woven polyethylene terephthalate and drawn out polytetrafluoroethylene prompted interest in thermoplastic elastomers for use as blood conduits. Medical grade polyurethane (PU) copolymers are an important member of the thermoplastic elastomer family. PU's are generally composed of short, alternating polydisperse blocks of soft and hard segment units. The soft segment is typically a polyester, polyether or a polyalkyldoil (e.g., polytetramethylene oxide). The hard segment is formed by polymerization of either an aliphatic or aromatic diisocyanate with chain extender (diamine or glycol). The resulting product containing the urethane or urea linkage is copolymerized with the soft segment to produce a variety of polyurethane formulations. PU's have been tested as blood conduits for over 30 years. Medical grade PU's, in general, have material properties that make it an excellent biomaterial for the manufacture of vascular grafts as compared to other commercial plastics. These properties include excellent tensile strength, flexibility, toughness, resistance to degradation and fatigue, as well as biocompatiblity. Unfortunately, despite these positive qualities, it became clear in the early 1980s that conventional ether-based polyurethane elastomers presented long-term biostabilty issues as well as some concern over potential carcinogenic degradation products. Further, in contrast to excellent performance in animal trials, clinically disappointing results with PU-based grafts diminished the attractiveness of the material for this application.

Recent developments in new generation polyurethanes, however, have made this biomaterial, once again,
a promising choice for a successful long-term vascular prosthesis. Specifically, the new generation of polyurethanes solved the biostabality problems but still provide clinically disappointing results. Poor performance is largely due to limitations of current manufacturing techniques that create a random or non-optimal fibrous structure for cell attachment using crude precipitation and/or filament manufacturing techniques. (U.S. Patents 4,173,689; 4,474,630; 5,132,066; 5,163,951; 5,756,035; 5,549,860; 5,863,627 & WO 00/30564)

Nonwoven or non-fibrous polyurethane vascular grafts have also been produced, and various techniques have been disclosed for swelling and/or gelling polyurethane polymers.

U. S. Patent No. 4,171,390 to Hilterhaus et al. discloses a process for preparing a filter material that can be used, for example, for filtering air or other gases, for filtering gases from high viscosity solutions, or for preparing partially permeable packaging materials. A first solution containing an isocyanate adduct dissolved in a highly polar organic solvent is admixed into a second solution containing a highly polar organic solvent and a hydrazine hydrate or the like. The first solution is admixed into the second solution over an extended period of time, during which time the viscosity of the admixture increases as the hydrazine (or the like) component reacts with the isocyanate to produce a polyurethane. The first solution is added up to the point of instantaneous gelling. The final admixture is coated onto a textile reinforcing material, and the coated material is placed in a water bath to coagulate the polyurethane. The resulting structure features a thin, poreless skin that must be removed, for example, by abrasion, if the structure is to be useful as a filter.

U.S. Patent No. 4,731,073 to Robinson discloses an arterial graft prosthesis comprises a first interior zone of a solid, segmented polyether-polyurethane material surrounded by a second zone of a porous, segmented polyether-polyurethane, and usually also a third zone surrounding the second zone and having a composition similar to the first zone. The zones are produced from the interior to the exterior zone by sequentially dipping a mandrel into the appropriate polymeric solution. The porous zone is prepared by adding particulates such as sodium chloride and/or sodium bicarbonate to the polymer resin to form a slurry. Once all of the zones have been formed on the mandrel, the coatings are dried, and then contacted to a water bath to remove the salt or bicarbonate particles.

U.S. Patent No. 5,462,704 to Chen et al. discloses a method for making a porous polyurethane vascular graft prosthesis that comprises coating a solvent type polyurethane resin over the outer surface of a cylindrical mandrel, then within 30 seconds of coating, placing the coated mandrel in a static coagulant for 2-12 hours to form a porous polyurethane tubing, and then placing the mandrel and surrounding tubing in a swelling agent for 5-60 minutes. After removing the tubing from the mandrel, the tubing is rinsed in a solution containing at least 80 weight percent ethanol for 5-120 minutes, followed by drying. The coagulant consists of water, ethanol and optionally, an aprotic solvent. The swelling agent consists of at least 90 percent ethanol. The resulting vascular graft prosthesis features an area porosity of 15-50 percent and a pore size of 1-30 micrometers.

U.S. Patent No. 5,977,223 to Ryan et al. discloses a technique for producing thin-walled elastomeric articles such as gloves and condoms. The method entails dipping a mandrel modeling the shape to be formed into a coagulant solution, then dipping the coagulant coated mandrel into an aqueous phase polyurethane dispersion, removing the mandrel from the dispersion, leaching out any residual coagulant or uncoagulated polymer, and finally curing the formed elastomeric article. When the polyurethane dispersion comprises by weight about 1 to 30 parts per hundred of a plasticizer based on the dry polyurethane weight, the dispersed polymethane particles swell. Thus, if the dispersion featured polyurethane particles having a mean size between 0.5 and 1.0 micrometer in the unplasticized condition, they might be between 1.5 and 3.0 micrometers in the plasticized condition. The inventors discovered that such swollen polyurethane particles produce a superior product, whereas in an unplasticized condition, particles of such a size (1.5-3.0 micrometers) impede uniform drying because of the large interstitial space between particles. Preferred coagulants are ionic coagulants such as quaternary ammonium salts; preferred plasticizers are the phthalate plasticizers.

U.S. Patent No. 5,229,045 discloses a technique for making porous membranes in tubular form that are suitable as implants, for example, small-diameter synthetic blood vessels. The membranes are formed by separately spraying a thermodynamically unstable polymer solution and a non-solvent onto a rotating surface such as a rotating mandrel. The polymer solution is made unstable by first dissolving polymer such as polyurethane or polysiloxane in a solvent, and then carefully adding a small quantity of non-solvent (but not so much as to cause coagulation or precipitation). The solvent is any that will dissolve the polymer, with tetrahydrofuran, amide solvents, sulfoxide solvents and dioxanes being preferred. The non-solvent may be any that is miscible with the solvent and has a solubility parameter different from the polymer, with water and the lower alkanols such as ethanol being preferred.

WO 00/55300 discloses a technique for making a three-dimensional porous scaffold for tissue culture, whereby the scaffolds may be molded in desirable forms and have desirable pore sizes and porosities. A polymer such as those based on lactic or glycolic acid is dissolved in a solvent, and some of the solvent is evaporated to make a solution of high viscosity. In an alternate embodiment, ethanol may be added to precipitate the polymer in a gel phase. An effervescent salt is added in a ratio of 1 to 100 times by weight of the polymer, and homogeneously mixed. The organic solvent is then removed, for example, by evaporation. Submergence of the polymer/salt gel slurry into hot aqueous solution or acidic solution effervesces the salt, resulting in a porous structure. The structure is then washed with distilled water, and then freeze-dried to yield the porous scaffold.

Methods using a precipitation/coagulation of polymer solutions by combining them with of non-solvents in order to produce materials useful as artificial leather are disclosed in the following:

US 3 492 154 A discloses a process for making a microporous sheet material, for example, for artificial leather applications. To make this material, a polymer is dissolved in a first solvent, and a slight amount of a non-solvent is added, but not enough to cause the polymer to precipitate out. The obtained solution is applied to a porous substrate, and then the dissolved polymer is coagulated by reducing temperature, for example, by immersing the substrate in a cold water bath. Solid, porous polymer precipitates out of solution, whereupon it may be washed and dried conventionally.

US 3 553 008 A is similar but leaves open the possibility (although not his preferred embodiment) of sufficiently coagulating his solution with the non-solvent to form a gel, and then working with the gel to make a a porous sheet. After shaping the solution or gel on a suitable substrate, the solvent and non-solvent are evaporated, leaving behind the porous polymer sheet. The addition of sufficient non-solvent to the solution causes the polymer to precipitate out of solution, thereby leaving a "serum" layer above or below a gelled, opaque, viscous mass.

The Abstract for Japanese Published Patent Application No. JP 51074058 A discloses a related technique for making a polymeric sheet that is gaspermeable. A solution is prepared, consisting of solvent containing polyurethane, or polymer prepared from a polyurethane base and a water-soluble high molecular weight polymer. The polyurethane is present in an amount of at least about six percent by weight, and any water-soluble polymer is present in an amount of about 0.5 to 4 percent by weight. To this is added a non-solvent for the polyurethane or high molecular weight polymer. This mixture is allowed to gel over a period of time. Then it is stirred to obtain a uniform polymer solution. The resulting solution is then coated on, or impregnated in, a fibrous base. The base is then treated with a solidifying solution containing the solvent of the water-soluble high molecular weight polymer and the non-solvent of the polyurethane. After drying, the resulting porous sheet is removed from the fibrous base.

US 3 527 653 A discloses another improvement in the manufacture of microporous artificial leather. Again, a polyurethane gel is coated or otherwise applied to a substrate and then coagulated. Instead of pure non-solvent, the hardening or coagulating agent is an aqueous solution containing electrolytes. Further, the electrolyte solution is provided at a pH between 3 and 9, and preferably at an electolyte concentration of at least 15% by weight, but possibly up to the saturation point. Metal salts such as sodium chloride or potassium nitrate are useful electrolytes. With this modification, the macropores are mostly eliminated, leaving only the (desired) micropores.

EP 0 382 356 A discloses an asymmetric membrane (small pores on one surface; larger pores on the other) featuring an at least partially crystalline aromatic ether ketone polymer. This polymer may be dissolved in strong acids that are substantially non-sulphonating reagents toward the polymer, such as methanesulphonic acid. Optionally, a pore-forming agent may be added at this point, such as an inorganic salt, a soluble amorphous organic polymer, or a low molecular weight organic compound. The solution is shaped to the desired shape, for example, by casting the polymer solution as a sheet on a non-reactive substrate to form a sheet membrane. The solution is then contacted with a non-solvent for the polymer, such as acetic acid, dilute sulphuric acid, water, methanol and ethanol, to precipitate the polymer. Once gelation of the polymer is substantially complete, it is rernoved from the non-solvent, and the residual strong acid/non-solvent is removed from the membrane by treatment with an aqueous medium.

The Abstract of Japanese Patent Publication JP 58 189242 A contains a similar teaching, but the polymer solution contains two or more polymer species, and one of them is extracted. In particular, Japanese Patent Publication JP 58 189242 A discloses leather substitutes with good softness and flexibility, and a method for making them. Specifically, composite fiber was spun from nylon 6 and polyethylene in a 1: 1 ratio, drawn, crimped, and formed into a needle-punched nonwoven sheet. This sheet was then impregnated with a 25% solution of polyurethane with poly(ethylene adipate) soft segments in DMF containing pore regulator. The impregnated sheet was then immersed in 40% aqueous DMF. to gel the impregnant, and then treated with hot toluene to extract the polyethylene, thereby making the substrate porous. The substrate was then buffed, and then coated with a 15% solution of various elastomers in DMF containing pore and gelatin regulators. The coated substrate was gelled in aqueous DMF to give the leather substitute.

The Abstract of Japanese Patent Publication JP 50 042004 discloses making a porous polymer having a high ratio of open cells to closed cells. The process begins with a composition featuring two polymers and a non-solvent. The second polymer is a compound that is soluble or swelled to both a good solvent for the first polymer, and is compatible with a substance that is a non-solvent for the first polymer. The composition is injected into a mold of porous material and gelled in the non-solvent or under an atmosphere of non-solvent vapor. The gel is then removed, and the second polymer is extracted and removed from the gel using the non-solvent (or non-solvent vapor). Preferred first polymers include polyurethane, polyvinylchloride, polystyrene, EVA and polyamide. Preferred second polymers include polyvinylalcohol, polyethylene oxide and methylcellulose. The good solvent is, for example, DMF, acetone, dioxane, tetrahydrofuran, or phenol. The non-solvent is for example, water or alcohol.

The Abstract of Japanese Patent Publication JP 2000 319434 A discloses a technique for making a high-porosity, large-pore diameter molded article in which a moldable polymer is dissolved in a solvent under heating, the polymer solution is gelled by treatment with a gelling agent, and the gel is then immersed in a poor solvent to extract the dissolving solvent. The polymer typically has a molecular weight of 50,000 to 500,000, and is exemplified by polystyrene, polymethyl methacrylate, polyethylene glycol, of polyethylene. The gelling agent is desirably one selected from an amino acid derivative and a cyclohexane diamine derivative. After the solvent is extracted, the gel is further immersed in a solvent to remove the gelling agent, also by extraction.

In each instance, more are severe shape-making limitations, e.g., the known non-fibrous methods appear to be limited to working with a relatively low viscosity liquid that can be coated onto a surface, or into which a shape-forming mandrel can be dipped. It would be desirable if the polymer could be rendered in the form of a gel because a gel, inter alia, can be molded. In other words, the gel can be plastically shaped and can retain its molded shape without reverting to its original shape. Usually the molded shape is preserved so that the shaped polymer retains the new shape and win return to the new shape if deformed, provided that the elastic limit is not exceeded. Further, most of the above-discussed non-fibrous art results in a product that features a non-porous layer at least at some location in the product. Thus, the prior art does not seem to appreciate the desirability of a prosthesis such as a vascular graft containing channels or porosity extending continuously from the exterior surface to the luminal surface of the graft.

One of the reasons for failure of vascular grafts is due to the formation of acute, spontaneous thrombosis, and chronic intimal hyperplasia. Thrombosis is initiated by platelets reacting with any non-endothelialized foreign substance, initiating a platelet agglomeration or plug. This plug continues to grow, resulting in occlusion of the graft. If the graft is not immediately occluded the plug functions as a cell matrix increasing the potential for rapid smooth muscle cell hyperplasia. Under normal circumstances, platelets circulate through the vascular system in a non-adherent state. The endothelial cells lining the vascular system accomplish this. These cells have several factors that contribute to their non-thrombogenic properties. These factors include, but are not limited to, negative surface charge, the heparin sulfate in their glycocalyx, the production and release of prostacylin, adenosine diphosphate, endothelium derived relaxing factor, and thrombomdulin. Thus, adherence of a thin layer of endothelial cells to the vascular prosthetic results in enhanced healing times and reduced failure rates of the graft.

Other reasons for artificial graft failure are neointima sloughing due to poor attachment and aneurysm formation resulting from compliance mismatch of the new graft material to the existing vascular system. It is important to know that materials with different mechanical properties, when joined together and placed in cyclic stress systems, exhibit different extensibilities. This mismatch may increase stress at the anastomotic site, as well as create flow disturbances and turbulence. Additionally, poor attachment geometry can lead to the problematic results above, due to flow disturbances and turbulence. For example, the harvesting of autograft veins typically causes a surgeon to use a graft of non-optimal diameter or length. A graft diameter mismatch, of perhaps 60% or more, causes a drastic reduction in flow diameter. Such flow disturbances may lead to para-anastomotic intimal hyperplasia, anastomotic aneurysms, and the acceleration of downstream atherosclerotic change.

Finally, artificial graft failures have been linked to leaking of blood through the device. Pre-clotting and the addition of short-lived bioabsorbable substances such as collagen, gelatin and albumin can prevent this as well as provide a matrix for host cell migration into the prosthesis. One problem with this approach is that the same open fibrous weave that permits blood leaking also allows the viscous bioabsorbable substances and clotted blood to accumulate on the luminal surface and easily detach resulting in complications (e.g., emboli) downstream from the device.

### DISCLOSURE OF THE INVENTION

The present invention manufactured through a novel gel enhanced phase separation technique solves the above listed problems that occur in existing vascular prostheses, both fibrous and non-fibrous.

According to the method of the present invention, a porous polymer is prepared by dissolving the polymer in a solvent and then adding a "gelling solvent". The "gelling solvent" for the polymer is not to be confused with a "non-solvent", which is a substance that causes the polymer to precipitate out of solution. The non-solvent is sometimes referred to interchangeably as the "coagulant" or the "failed solvent". Unless indicated otherwise, for purposes of this invention, the solvent that dissolves the polymer is interchangeably referred to as the first solvent, and the gelling solvent is interchangeably referred to as the second solvent.

Significantly, when a "gelling solvent" is added to a polymer/solvent solution the polymer does not precipitate out as it would with a "non-solvent". Instead, the entire volume begins to thicken as the dissolved polymer absorbs the "gelling solvent". As more "gelling solvent" is added, the viscosity of the entire volume increases to the point where it becomes a gelatinous mass that can be picked up, e.g., a stable gel. This gel can then be spread out onto plates or transferred into molds. The plates or molds can then be immersed into a non-solvent that leaches the original solvent from the gel or placed under vacuum to pull the solvent from the gel, leaving an intercommunicating porous network. The unit is then cured for several hours in an oven to permanently set the architecture. Varying the concentration of polymer in the first solution and/or the concentration of the "gelling solvent" added will reproducibly alter the porosity. Polymers useful for the creation of the finished article (e.g., a tubular prosthesis) include but are not limited to the following groups: a) polyurethanes; b) polyureas; c) polyethylenes; d) polyesters; and e) fluoropolymers.

The articles created using this technique include, but are not limited to, a non-metallic, non-woven, highly porous graft material having an inner surface and an outer surface, and having a plurality of openings throughout its bulk providing a highly convoluted intercommunicating network of chambers between its two surfaces, the walls of the chambers providing a large surface area. In part, it is this highly porous, convoluted intercommunicating network of chambers that allows the present invention to overcome problems that have plagued previous vascular grafts.

The creation of a stable gel that can be injected into finely detailed molds without risk of clumping of the precipitate or salt, is a vast improvement over existing technologies. This gel will open up the possibility of mass production of complex prostheses, including heart valve, bladder, intestinal, esophagus, urethra, veins and arteries, via an automated system. Additionally, articles produced through the practice of this invention include larger components, with complicated geometries, and unique density-property-processing relationships; of which, these articles may be used in various industries (e.g., automotive, consumer goods, sporting goods, etc.).

### BRIEF DESCRIPTION OF THE DRAWINGS

- FIGS. 1-10: are Scanning Electron Microscope (SEM) images of four different vascular grafts made from four different species of polymer using the gel enhanced phase separation technique;
- FIG.11: is an optical photograph showing a pattern of tissue invasion into the porosity of the graft;
- FIG 12: is a schematic illustration of the polymeric microstructure in the prior vascular grafts (right drawing) versus the polymeric microstructure in the vascular grafts of the present invention (left);
- FIGS. 13a-13c: show a possible embodiment of the present invention allowing for improved suturing; and
- FIGS. 14a-14d: show various embodiments of the present invention made possible by the gel enhanced phase separation technique.

### MODES FOR CARRYING OUT THE INVENTION

While working with several different species of polymer, a new and unique method for controlled incorporation of intercommunicating pores within the polymers was discovered. In a preferred embodiment, the method for preparing the porous polymers involves dissolving the polymer in a solvent and then adding a "gelling solvent". The "gelling solvent" for the particular polymer is not to be confused with a "non-solvent" that causes the polymer to precipitate out of solution. Solid polymer particles placed in contact with a gelling solvent swell as they absorb the gelling solvent and take on fluid like properties but do not loose cohesiveness and remain as discrete, albeit swollen particles.

A common example of this phenomenon exists in the polymers used to make soft contact lenses. Hydroxyethylemethacrylate (HEMA) can achieve water contents ranging from 35% to 75% when immersed. The water is absorbed into this solid brittle polymer and transforms it into a swollen soft mass. Water functions as a "gelling solvent" for this polymer.

When a "gelling solvent" is added to a polymer/solvent solution, the polymer does not precipitate out as it would with a "non-solvent". Instead, the entire volume begins to thicken as the dissolved polymer absorbs the "gelling solvent". As more "gelling solvent" is added the whole mass turns into a gelatinous mass that can be picked up. If the beginning polymer/solvent volume was 20ml, and 20ml of "gelling solvent" were added, the result would be 40ml of gel. This gel can then be shape-formed, e.g., molded, for example, by spreading or injecting the gel over a plate or a three-dimensional object, or by forcing a plate or three-dimensional object into the gel. The plates or molds can then be immersed into a non-solvent that leaches the original solvent from the gel. Alternatively, the plates or molds may be placed under vacuum to pull the solvent from the gel, leaving an intercommunicating porous network. The unit is then cured for several hours in an oven to permanently set the architecture. (In most cases the gelling agent is also removed in the leaching or vacuum process.) Varying the polymer concentration in the original solution and/or varying the concentration of the "gelling solvent" added will reproducibly alter the porosity. For example, the lower the concentration of polymer, the more porous is the final product. Polymers useful for the creation of the final article include but are not limited to the following groups: a) polyurethanes; b) polyureas; c) polyethylenes; d) polyesters; and e) fluoropolymers.

The articles created using the techniques of the current invention include a non-metallic, non-woven highly porous graft material having a plurality of openings throughout its substance providing a highly convoluted intercommunicating network of chambers between its two surfaces, the walls of the chambers providing a large surface area. In part, it is this highly porous, convoluted intercommunicating network of chambers that allows the present invention to overcome problems that have plagued previous vascular grafts, and further offers unique properties useful to the various aforementioned industries and product types.

Similar appearing technologies that utilize simple phase separation/precipitation in non-solvents or leaching of solid particles such as salt are difficult if not impossible to reproduce on a large scale due to their demand for constant skilled human interaction. Additionally they are limited in the final conformation of medical device formed. The creation of a stable gel, which can be injected into finely detailed molds without risk of clumping of the precipitate or salt, is a vast improvement over existing technologies. This gel will open up the possibility of mass production of complex articles such as, for example, prostheses, including heart valve, bladder, intestinal, esophagus, urethra, veins and arteries, via an automated system. A specially designed press can be used for injection of the gel into custom molds containing wings, flaps, ribs, waves, multiple conduits, appendages or other complex structures unavailable to prior art devices. The molds will then move to an immersion and/or vacuum chamber to remove the dissolving solvent and "gelling solvent", after which the devices are placed into a curing oven.

Composite or multifaceted materials can be fabricated by placing the gel in contact with one or more other materials. Examples of such other materials include, but are not limited to, biologically active agents, and biodegradable or non-degradable sutures or fibers and reinforcement rings. The gel could be, for example, injected over a suture, or injected into a mass of fibers. Additionally, two different gels composed of different polymer concentrations or polymers can be layered on top of or mixed with each other to create laminates and composites previously unknown. At this point, at least the gel portion of the resulting mass is still shapeable (e.g., moldable), and accordingly can be shaped by known techniques to the desired geometry. The solvent is then removed as described previously, leaving the porous polymer material and the other material mechanically attached to one another. The resulting composite body could represent the entire article, or it could be merely a component of a larger article (e.g., an entire prosthesis or simply a component thereof).

As suggested by the above embodiment of injecting the gel into a mass of fibers, one or more reinforcement materials (e.g., particulate, fibers, whiskers, woven materials, etc.) may be incorporated or admixed with the present polymers by known techniques. A very typical reason for incorporating such a reinforcement (but by no means the sole reason) is to enhance certain physical properties such as strength, stiffness, etc.

In the prosthesis embodiment of the invention, it is the intent to allow uninterrupted tissue connection, e.g., contiguous tissue, to exist throughout the entire volume of the prosthesis. Thus when a neointima forms across the lumen of the prosthesis, it is not only attached to the surface of the graft material, but additionally anchored to the tissue growing through the prosthesis. Once fully integrated with tissue, the graft is hidden by the newly formed endothelial cell lining from the blood flowing through it and thus benefits from the endothelial cells' non-thrombogenic properties.

Additionally, the material produced by this preferred teaching of the present invention may occupy only a small fraction of the overall volume of the device. This allows the tissue within the device to dictate the mechanical properties of the device preventing a compliance mismatch of the graft material to the existing vascular system.

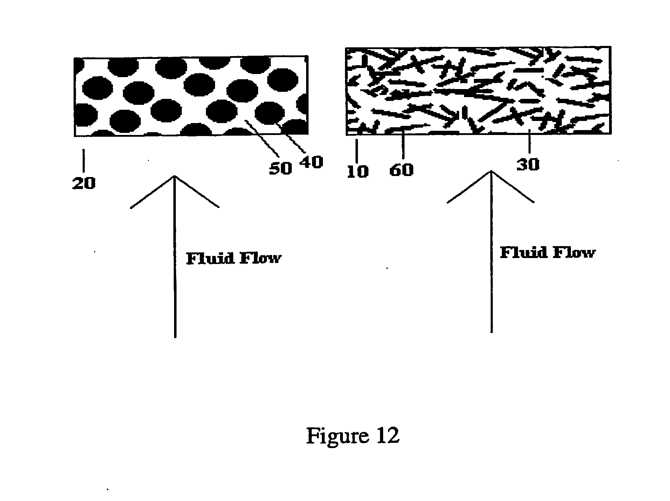

Finally, the unique arrangement of intercommunicating chambers within the device prevents leaking of blood through the device by slowing the movement of blood through the thickness of the unit many times over, allowing it to clot and self-seal. The fibrous structure in state of the art grafts provides rounded cylinders throughout the mass of the device (see Figure 12, left side). These cylinders provide a low surface area and thus relatively low resistance to flow. To compensate for this, the density of fibers must be increased, reducing the overall porosity of the unit. The present invention overcomes this by providing thin flat plates of polymer material having a relatively large surface area to disrupt flow through the chambers defined by the flat surfaces (Figure 12, right). The large surface area of each individual chamber slows the movement of blood, creating small interconnecting clots. These clots are then trapped within the internal chambers of device and cannot be sloughed off into the blood stream.

In another aspect of the present invention, other bioabsorbable substances can be impregnated into the chambers of the device and be protected from the circulating blood. For example, it may be beneficial to incorporate the bioabsorbable substance into the chambers as a liquid and freeze-dry it to form a microstructure. This microstructure, particularly if it is soluble in tissue fluids, can then be cross-linked or in some other way stabilized so that it typically must be degraded to be removed from the prosthesis. Incorporation of the stabilized microstructure can then be used to fine-tune the properties of the graft to that of the host vessel. The purpose of the microstructure is at least four-fold: (i) provide a temporary pore seal to further increase resistance to flow through the thickness of the unit; (ii) increase the biocompatibilty of the overall prosthesis for cellular attraction and attachment; (iii) provide for control of mechanical properties other than via concentration of constituents of the gel-enhanced phase separation process; and (iv) provide a medium for the delivery of biologically active agents to, for example, mediate or moderate the host response to the implant graft.

Useful bioabsorbable substances include collagen, gelatin, succinylated collagen, chondroitin sulfate, succinylated gelatin, chitin, chitosan, cellulose, fibrin, albumin, alginic acid, heparin, heparan sulfate, dermatin sulfate, keratan sulfate, hyaluronic acid, termatan sulfate, polymerized alpha hydroxy acids, polymerized hydroxy aliphatic carboxylic acids, polymerized glycolic acids and derivatives of these members.

Representing yet another important aspect of the present invention, an additional benefit of the microstructure isolation within the intercommunicating chambers is the ability to carry and retain one or more biologically active agents within the article or prosthesis. The biologically active agents can promote healing and tissue invasion, and are protected from the flowing blood. These biologically active agents include physiologically acceptable drugs, surfactants, ceramics, hydroxyapatites, tricalciumphosphates, antithrombogenic agents, antibiotics, biologic modifiers, glycosaminoglycans, proteins, hormones, antigens, viruses, cells and cellular components. The biologically active agent can be added to the microstructure before or after cross-linking. Moreover, the biologically active agent can be added during the gel enhanced phase separation process for producing the porous polymeric material. For example, the biologically active agent can be mixed with the polymer and first solvent prior to addition of the gelling solvent; it can be mixed with the gelling solvent prior to addition of the gelling solvent to the polymer/first solvent solution; or it can be mixed with the gel prior to removal of the solvents. Still further, the biologically active agent can be incorporated within the pores of the polymeric material after removal of the solvents.

Among the non-limiting advantages of using the present non-woven architectured synthetic implant instead of autograft or allograft as vascular grafts are the following:
1, sterile off-the-shelf implant;
2. availability of multiple diameter and length implants;
3. can be molded into unique shapes and designs to improve handling characteristics;
4. lowered risk of aneurysm;
5. no risk of disease transmission;
6. allows for easy ingrowth of fibrous tissue, which stabilizes and anchors the implant.
7. allows for vascular ingrowth (vasa vasorum) nourishing the graft and providing access to free floating stem cells.
8. the graft is straight, flexible and can be twisted in any direction. (This is a major advantage over autografts and allografts that must be implanted in their original shape to avoid complications.)
9. allows for incorporation ofbioabsorbable substances to improve biocompatability.
10. allows for incorporation of biologically active agents to aid in healing.
11. can be fabricated to have varying physical, chemical and mechanical properties along its length.

Among the non-limiting advantages of using the present non-woven architectured synthetic implant instead of present state-of-the-art woven or fibrous implants are the following:
1. interpenetrating pore structure allows for rapid but stable cellular ingrowth;
2. can be molded into unique shapes and designs to improve handling characteristics;
3. pore structure with large surface area reduces hemorrhage through the implant;
4. use of stabilized microstructure allows use of device with larger pore structure without hemorrhage risk;
5. creation of a living tissue barrier protects the material of the implant from coming in direct contact with blood flowing through the lumen;
6. allows for easy ingrowth of fibrous tissue which stabilizes and anchors the implant;
7. unbroken weave of tissue throughout device distributes stresses in an optimal manner, reducing occurrence of compliance mismatches.
8. allows for vascular ingrowth (vasa vasorum) which nourishes the graft and provides access to free floating stem cells.
9. pore structure allows the device to carry bioabsorbable materials without loss to circulatory system.
10. pore structure allows the device to support biologically active agents without dilution or loss to circulatory system.
11. use of flat plates provides a greater surface area using less material allowing for a higher overall porosity.

Among the medical application areas envisioned for articles produced in accordance with the various teachings of the present invention include, but are not limited to, prostheses for use in vascular reconstructive surgery of mammals, including humans and other primates. The prosthesis may be used to repair, replace or augment a diseased or defective vein or artery of the body. The prosthesis may also be used as a substitute for the ureter, bile duct, esophagus, trachea, bladder, intestine and other hollow tissues and organs of the body. Additionally, the prosthesis may function as a tissue conduit, or, in sheet form it may function as a patch or repair device for damaged or diseased tissues. (e.g., heart, heart valves, pericardium, veins, arteries, stomach, intestine, bladder, etc.) When functioning as tissue conduit (e.g., nervous tissue) the lumen of the prosthesis may also carry substances that aid in tissue growth and healing.

In a preferred embodiment of the present prosthesis invention, namely that of a vascular graft, the graft consists of a polyurethane conduit composed of small chambers with each chamber being formed of multiple thin flat partitions. The thickness of each polymer partition is only a fraction of its length and height This allows a small mass of polymer to create a large surface area providing high resistance to blood flow through the thickness of the prosthesis. One chief disadvantage of a highly porous vascular graft is its high permeability to blood during implantation leading to blood leakage through the graft wall. The unique arrangement of the intercommunicating chambers within the device of the present invention, however, prevents the leaking of blood by drastically slowing its movement through the thickness of the graft and allowing it to clot and self-seal.

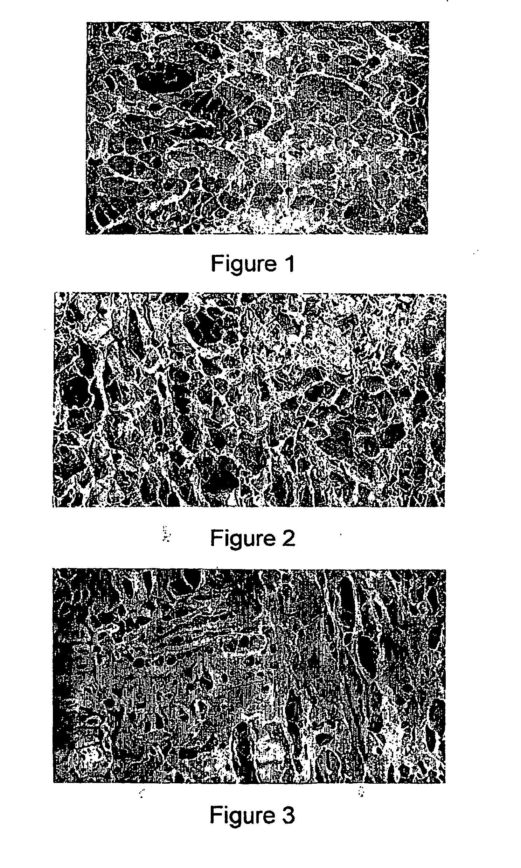

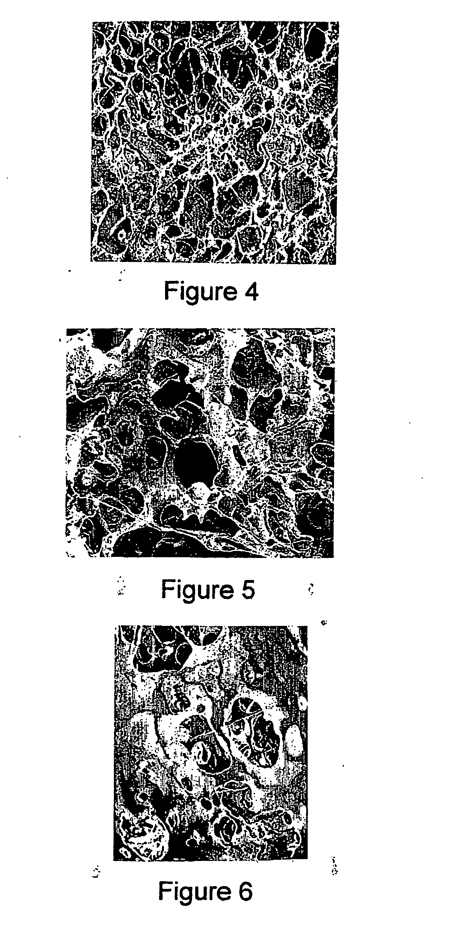

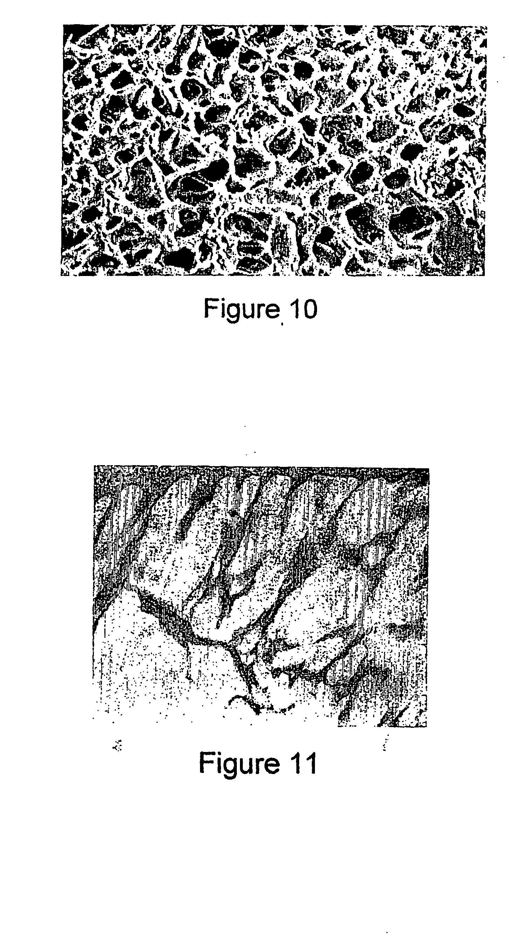

Referring now to the figures, those of Figures 1-10 illustrate Scanning Electron Microscope (SEM) images of four different vascular grafts made from four different species of polymer using the gel-enhanced phase separation technique. In particular, Figures 1, 4 and 7 are SEM images, taken at 250X, 240X and 260X magnification, respectively, showing the external graft surface using a siloxane polyurethane polymer, a carbonate polyurethane polymer, and a resorbable lactic acid polymer. The external surfaces have a high overall porosity. In contrast, the luminal sides of the grafts have a smooth, low pore surface to minimize flow disturbances. See, for example, Figures 3, 6 and 9, which are SEM images at 250X magnification of the luminal surface of vascular grafts made from the siloxane polyurethane polymer, the carbonate polyurethane polymer, and the resorbable lactic acid polymer, respectively. Figures 2, 5 and 8 are the corresponding SEM images through the cross-section of the above-mentioned polyurethane and lactic acid polymer grafts, but taken at magnifications of 250X, 260X and 150X, respectively. Figure 10 is a 250X magnification SEM image of a cross-section of a vascular graft made from a non-resorbable Teflon^{®} polymer. This area of the prosthesis provides multiple chambers capable of carrying other substances and provides a high surface area for cellular attachment while resisting flow through the graft.

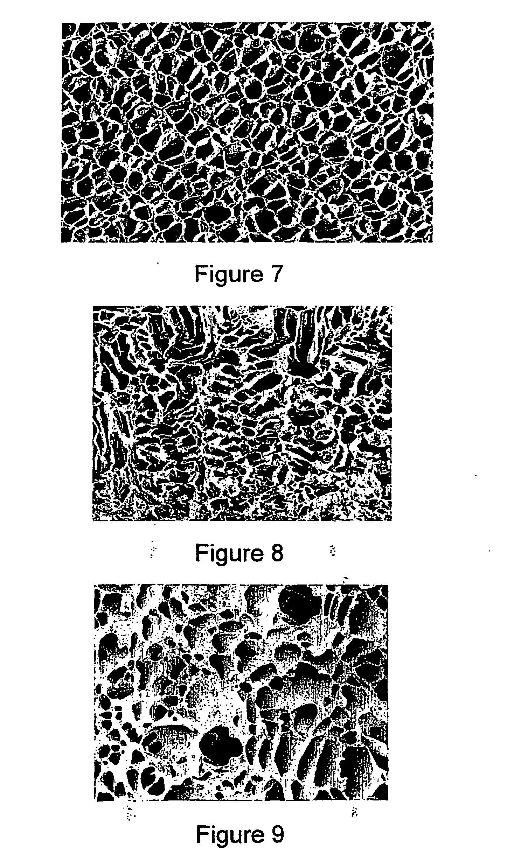

The speed and extent of peripheral tissue ingrowth determines the long-term compliance of the graft. Figure 11 is a 100X magnification optical photomicrograph showing fronds of tissue growing into the pores of a porous prosthesis and expanding to form an intercommunicating tissue network. The type, size and density of the pores of the vascular graft of the present invention not only affects the speed and extent of peripheral tissue ingrowth, but also influences the development and stability of an intimal endothelial layer. Upon implantation, the graft surface in contact with the host tissue bed typically is of a higher overall pore density so that tissue can quickly grow into the prosthesis and secure it (compare, for example, Figure 7 with Figure 8). In contrast, the luminal surface of the graft usually has a smooth, low pore density surface in contact with blood to minimize flow disturbances. Not entirely without intercommunication, the luminal surface of the conduit does present enough porosity so that the new cellular lining can be anchored to the tissue that has grown into the device (compare, for example, Figure 9 with Figure 8). The pore size ranges from about 20 to about 75 microns in diameter.

Present commercially available vascular prostheses fail to form a complete endothelial lining. At best they have an anastomotic pannus formation that rarely achieves 2 cm in length. To achieve long-term patancy, a prosthesis probably will require complete endothelialization, and such can only be supported if there is full micro-vessel invasion from the surrounding connective tissue into the interstices of the prosthetic device, nourishing the neointima. Accordingly, in the second aspect of the present invention, where a secondary bioresorbable "microstructure" material is incorporated into the interstices of the polyurethane graft "macrostructure", such investment of the secondary bioresorbable material can encourage the formation of the complete endothelial layer, e.g., by allowing for ingrowth of collateral circulation to nourish the cells within the prosthesis.

Materials such as collagen gels have been utilized for years to avoid pre-clotting of vascular grafts and to improve biocompatability of the implant. Due to the high solubility of these materials, their benefits are short lived. Within a matter of hours these gels are stripped out leaving the prosthesis nude. Several hours may provide sufficient time to avoid pre-clotting, but is not adequate to aid in tissue integration. In response to the foreign material the body forms a dense tissue capsule over the external surface of the graft. This capsule prevents infiltration of micro vessels through the prosthesis necessary to stabilize an endothelial layer on the luminal surface.

In contrast, and in a particularly preferred embodiment of the present invention, the pore structure of the present prosthesis accommodates and protects the collagen gel (refer again to Figure 12). Additionally, once incorporated, the gel may be lyophilized and cross-linked. Preferably the cross-linking will be accomplished by a di-hydrothermal technique that does not require the use of toxic chemicals. The pore structure and cross-linking should allow the gel to remain within the pore structure of the graft for several days, instead of hours. This additional time should be sufficient to encourage cells to enter the device and attach to each polymer partition making up the graft, forming a living tissue barrier between the material of the graft and host cells and body fluids. Micro vessels are now free to grow from the external tissue bed, between the individually encapsulated polymer partitions, where they can stabilize a luminal endothelial layer. During that time between implantation and cellular invasion, the microstructure will provide increased resistance to fluid leakage and influence the biomechancial properties. In this way a more compliant macrostructure can be implanted which possesses characteristics that can be tailored to those of the host vessel by the physical properties of the microstructure. Specifically, the porous polymeric material is very compliant, and if the porous polymeric material ends up being more compliant than the tissue to which it is to be grafted, the secondary bioabsorbable material can reduce the overall compliance of the prosthesis to approximately that of the host tissue. Over time, host cells, which dictate the overall compliance of the graft, replace the microstructure.

Additionally, the di-hydrothermally cross-linked microstructure provides a larger window of time for utilization of biologically active agents than would exist for the gel alone. Growth factors can be retained within the boundaries of the prosthesis for an extended period of time where they can influence cells entering the device. The effective lifetime of anti-coagulants can be extended, providing additional protection until endothelialization occurs.

A different approach to promotion of capillary endothelialization through the walls of the vascular graft is disclosed in U.S. Patent No. 5,744,515 to Clapper. Specifically, the graft is sufficiently porous to allow capillary endothelialization, and features near at least the exterior wall of the graft a coating of tenaciously bound adhesion molecules that promote the ingrowth of endothelial cells into the porosity of the graft material. The adhesion molecules are typically large proteins, carbohydrates or glycoproteins, and include laminin, fibronectin, collagen, vitronectin and tenascin. Clapper states that the adhesion molecules are supplied in a quantity or density of at most only about 1-10 monolayers on the surface of the graft, and specifically on the pore surface. Thus, unlike the present secondary bioabsorbable materials, the adhesion molecules of Clapper seemingly would have a negligible effect on, for example, tailoring the mechanical characteristics of the graft, e.g., mechanical compliance.

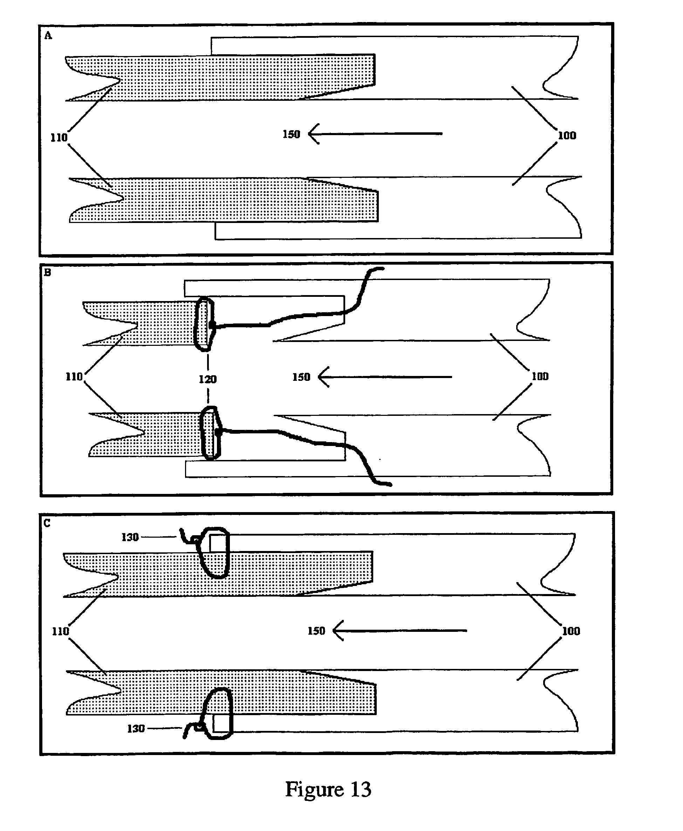

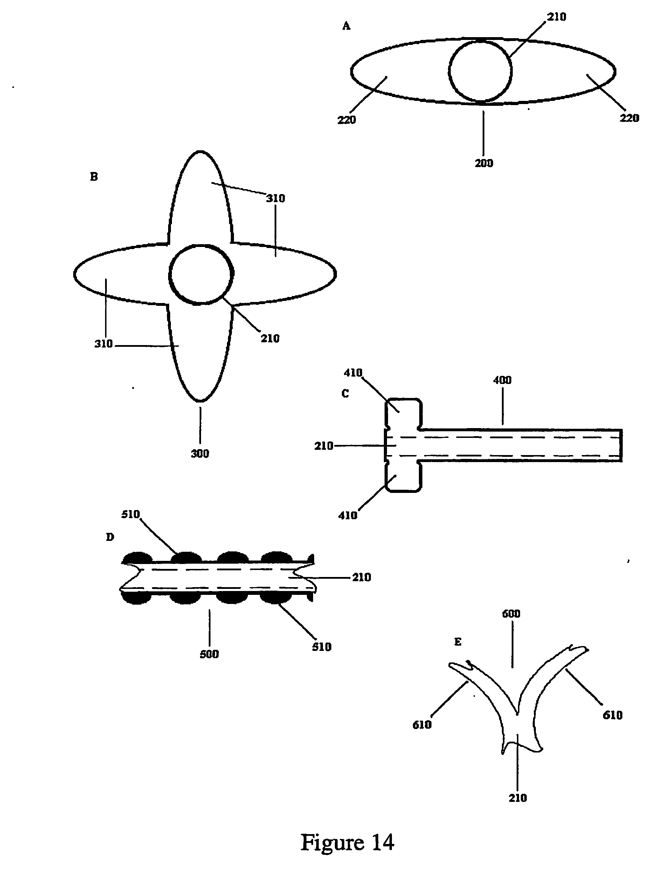

Again, one of the primary application areas envisioned for the present invention includes a prosthesis for use in vascular reconstructive surgery of mammals, including humans and other primates. The prosthesis may be used to repair, replace or augment a diseased or defective vein or artery of the body. Figure 13, for example, shows a possible embodiment of the present invention allowing for improved suturing. Specifically, Figure 13a shows how the host vessel, situated into the graft material, provides less resistance to flow through the lumen. Figures 13b and 13c show how sutures can be placed so that they do not encroach upon the lumen, thus minimizing flow disturbances. Figure 14 shows a representative, but non-limiting selection of various physical or structural embodiments of the present invention made possible by use of the gel-enhanced phase separation technique. For example, Figure 14a is an end-on view of a vascular graft showing that the present vascular graft may be provided with a pair of flaps to prevent rolling of the graft once implanted The vascular graft of Figure 14b provides additional support when compared to Figure 14a, namely, by providing two pairs of flaps. Figure 14c illustrates a graft with wings to facilitate suturing. Figure 14d is a view of a longitudinal section through a graft showing reinforcement rings around the circumference of the graft. Figure 14e depicts a "Y" graft used to split the blood flow.

The "Y" graft, or branched geometry is particularly useful to the vascular graft embodiment, as well as others, and this and other synthetic grafts may be attached by a port, connector or anastomosis, to an artery, vein, or other tubular or hollow body organ to effect a shunt, bypass, or to create other access to same. Additionally, a graft or other device produced with this invention may comprise a plurality of branches, with each branch having a length or diameter that may vary independently from the other branches. As an example, the inlet or proximal branch may be large, and attached to the large section of aorta, while distal sections may be significantly smaller, and of different lengths, to facilitate attachment to smaller coronary arteries.

The large proximal section could allow adequate blood flow through a single attachment to the aorta, thereby decrease possibility of leakage at various proximal anastomoses, while decreasing the procedural time. Likewise, diametric and length matches, or closer matches, will allow faster and easier connections; since the surgeon can trim the graft section to the appropriate length, and the surgeon will not have to rework the graft material to allow the larger natural vein to connect with the smaller coronary artery, thereby further decreasing procedure time.

This process will allow the graft to be of decreasing diameter with increasing length, thereby approximating the anatomy of the coronary artery system This allows the surgeon to trim the graft to any length, while maintaining a constant graft-vein diameter ratio, thereby allowing in situ customization of the graft length without incurring turbulent flow due to diameter mismatch.

In addition to facilitating the procedure, by reducing the duration of the surgical procedure and attachment complexity thereof, the diameter tailoring of this embodiment will allow the maintenance of a constant flow velocity, while the volume decreases (following the branches, each of which reduce the flow). This constant velocity is important to keeping blood-bome material in the mix; that is, plaque deposits may be deposited on the arterial wall or bifurcation junctions (e.g., the ostium) in the coronary system, in natural as well as in the synthetic graft.

The tailorable properties of material manufactured by the processes of this current invention allow for the manufacture of grafts and other vascular prostheses that may demonstrate flexibilities and expansion, under normal or elevated blood pressures, similar to that of natural arteries. This constraint-matching avoids problems associated with existing grafts, that is, these grafts and prostheses readily expand during the systolic pulsing. Grafts or harvested veins that do not expand can cause spikes in blood pressure, and may cause or exacerbate existing problems, including or due to high blood pressures.

The unique characteristics of the many polymer species available, both now as well as those anticipated in the future, make it impractical to provide a comprehensive list of gelling solvents. To address this problem, below is provided an example of a step-by-step process for the identification of useful dissolving solvents and gelling solvents for a single polymer species, as well as how the solvents may be removed to provide the porous, solid polymer material. This process example provides guidance in how to utilize the information provided in this disclosure; however it is recognized that alternate selection methods and/or criteria are known to those skilled in The art.

### EXAMPLE

A siloxane-based macrodiol, aromatic polyurethane, supplied by Aortech Biomaterials, was selected for this example.
1) The manufacturer identified dimethyl acetimide, n-methyl pyrrolidinone, and tetrahydrofuran as solvents for the polymer.
2) A 0.25-gram sample of polymer was placed into the bottom of 20 small bottles. Five milliliters of 20 common laboratory solvents, including the three listed by the manufacturer, was added to the bottles. The bottles were left for 48 hours at room temperature after which they were used to identify those solvents that dissolved or resulted in swelling of the polymer. Twelve solvents were identified and are listed below along with freezing point ("F.P.", also known as melt point), boiling point ("B.P."), vapor pressure ("V.P.''), and solvent group (S.G.). (Other properties that can aid in the selection of solvent and gelling solvent include, but are not limited to, density, molecular weight, refractive index, dielectric constant, polarity index, viscosity, surface tension, solubility in water, solubility in alcohol(s), residue, and purity.)

| | Contents | F.P. | B.P. | V.P. (torr) | S.G. | Result |
|---|---|---|---|---|---|---|
| 2 | acetone | -94.7 | 56.3 | 184.5@20C | 6 | swell |
| 5 | chloroform | -63.6 | 61.2 | 158.4@20C | 8 | swell |
| 7 | p-dioxane | 11.8 | 101.3 | 29.0@20C | 6 | swell |
| 11 | methylene chloride | -95.1 | 39.8 | 436.0@25C | 5 | swell |
| 12 | n,n-dimethyl acetimide | -20.0 | 166.1 | 1.3 @25C | 3 | dissolve |
| 13 | dimethyl sulfoxide | 18.5 | 189.0 | 0.6 @25C | 3 | swell |
| 14 | 1-methyl-2-pyrrolidone | -24.4 | 202.0 | 4.0@60C | 3 | dissolve |
| 15 | Tetrahydrofuran | -108.5 | 66.0 | 142.0@20C | 3 | dissolve |
| 16 | toluene | -95.0 | 110.6 | 28.5@20C | 7 | swell |
| 17 | m-xylene | -47.7 | 139.3 | 6.0@20C | 7 | swell |
| 18 | o-xylene | -25.2 | 144.4 | 6.6 @25C | 7 | swell |
| 20 | methyl-ethyl-ketone | -86.7 | 79.6 | 90.6@20C | 6 | swell |

3) From the chart, Tetrahydrofuran (THF) was selected as the polymer dissolving solvent due to its low freeze point, low boiling point and high vapor pressure. The skilled artisan can see that, for this particular polymer, solvent group #3 is particularly preferred as the dissolving solvent, and that solvent group #6 and group #7 are particularly preferred as the gelling solvent. The chart also shows that certain solvents from solvent group #5 and group #8 also gave a positive result, e.g., swelling, but these solvents were in the minority; the majority of solvents from these groups neither dissolved nor swelled the polyurethane. Accordingly, this information can be used to prioritize a search for other suitable solvents.
4) Five milliliters of a 12.5% solution of polymer and THF was placed into each of 9 small flasks with a magnetic stir bar at the bottom. Twenty milliliters of one of each of the 9 solvents identified as gelling agents was added to each flask with rapid stirring. After 2 minutes, stirring was stopped and the solutions were allowed to sit for 13 minutes. As expected, none of the additions resulted in precipitation of the polymer. As a control an additional flask was set up and 20 ml of ethanol (e.g., a failed solvent) was added with rapid stirring. A white precipitate immediately formed. After stirring was stopped the polymer precipitate drifted to the bottom of the flask.
5) All 9 flasks showed signs of thickening even though the polymer to solvent concentration fell from 12.5% to 2.5%. (The control flask solvents (20ml ethanol 5-ml THF/Polymer) became less viscous as the polymer fell out of solution.) Other parameters being kept equal, the viscosity of the resulting solution or mixture, upon adding the gelling solvent, increases with increasing concentration of polymer and increasing concentrations of gelling solvent. The viscosity also depends on the identity of the gelling solvent, and can range from a slight thickening to the formation of a gelatinous solid. At the concentrations listed, p-dioxane, dimethyl sulfoxide, and o-xylene produced the greatest thickening.
6) Utilizing the information provided in the chart, the following methods were used to remove the solvent and gelling agent:

### Sample A

Recognizing that p-dioxane has a freeze point, boiling point and vapor pressure suitable for freeze-drying; the Vial 7 gel was scooped onto a Teflon plate, spread out and frozen. The frozen gel (-15C) was then placed into a freeze-dryer for 12 hours. The THF, having such a low boiling point and high vapor pressure most likely does not freeze and thus is removed from the system first. Upon subsequently removing the p-dioxane, a white porous sheet was produced with a non-fibrous porosity greater than 90%.

### Sample B

Recognizing that dimethyl sulfoxide has a boiling point and vapor pressure unsuitable for freeze-drying, the Vial 13 gel is instead poured onto a Teflon tray, frozen at-15C and then submerged into a non-solvent (ethanol) at-10C for 12 hours to leach out the solvent and gelling solvent. (Had the gel been thick enough to form a stable gelatinous mass, freezing and the use of chilled alcohol would not be required.) The sheet was then removed form the alcohol and soaked in distilled water 12 hours, after which it is dried and placed into a desiccator. The sheet formed was relatively stiff and had a non-fibrous porosity of greater that 75%.

### Sample C

Comparing the boiling point and vapor pressure of o-xylene and THF the skilled artisan can see that it would be possible to heat the gel and selectively remove the THF solvent and leave the o-xylene gelling agent behind. Accordingly, the Vial 18 gel was poured into a Teflon dish and slowly heated from 21C to 66C over a 3-hour period. This increased the viscosity to that of a non-flowing gel without mechanical competence. The dish was then lowered into a 21C-ethanol bath for 12 hours to remove the o-xylene and any residual THF. A light tan sheet was produced with a non-fibrous porosity greater than 40%.

### COMPARATIVE EXAMPLE

Instead of first dissolving the polyurethane in the THF, an attempt was made to dissolve the polyurethane in a solution of THF and gelling solvent provided in the same ratio as in the Example. The polyurethane did not dissolve.

Thus, the Example and Comparative Example show: (1) that in the polyurethane/THF system, ethanol is a failed solvent that causes polyurethane to precipitate; (2) that the polymer preferably is dissolved before being exposed to the gelling solvent; (3) that different gelling solvents affect the solution viscosity to a different degree; and (4) that there are different ways to precipitate the porous polymer from solution, and that the preferred technique may depend upon the properties of the dissolving solvent and gelling solvent.

Having taught the reasoning process that is used in choosing appropriate first and second solvents for a given polymer, and appropriate techniques for their removal once a desired shape has been fabricated, an artisan of ordinary skill can readily identify without undue experimentation other polymer/first solvent/second solvent systems that can be processed similarly to what has been described herein to produce porous polymeric bodies. Accordingly, the artisan of ordinary skill will readily appreciate that numerous modifications may be made to what has been described above without departing from the claimed invention, the scope of which is set forth in the claims to follow.

## Claims

1. A process for creating a porous polymeric body of desired shape, comprising the steps of
a. selecting a polymer;
b. dissolving the polymer in a suitable first solvent to form a solution;
c. adding a quantity of a suitable second solvent to the solution, whereupon an entire volume of the solution begins to thicken into a gel;
d. continuing the adding of the second solvent until a viscosity of the gel increases to a point where the gel is suitable for shape-forming;
e. shape-forming the gel; and
f. removing the first and second solvents from the gel, thereby leaving an intercommunicating porous network.

2. The process of claim 1, further comprising placing the gel in contact with at least one other material whereby upon removing the first and second solvent the porous polymer and the at least one other material are mechanically bound to each other.

3. The process of claim 1, wherein the polymer comprises at least one polymer selected from the group consisting of polyurethanes, polyureas, polyethylenes, polyesters and fluoropolymers.

4. The process of claim 1, wherein said first solvent comprises an organic solvent selected from the group consisting of n,n-dimethyl acetimide, 1-methyl-2-pyrrolidone, and tetrahydrofuran.

5. The process of claim 1, wherein said second solvent comprises an organic solvent selected from the group consisting of acetone, chloroform, p-dioxane, methylene chloride, dimethyl sulfoxide, toluene, m-xylene, o-xylene, and methyl-ethyl-ketone.

6. The process of claim 1, wherein said first solvent comprises an organic solvent selected from the group consisting of Solvent Group 3.

7. The process of claim 1, wherein said second solvent comprises at least one organic solvent selected from the group consisting of Solvent Group 6 or Solvent Group 7.

8. The process of claim 1, wherein forming of the gel comprises at least one technique selected from the group consisting of
(i) spreading the gel onto an open smooth or textured surface;
(ii) injecting the gel into a mold;
(iii) spreading or injecting the gel over a three-dimensional object, and removing the three-dimensional object after removing the first and second solvent from the gel; or
(iv) forcing a three-dimensional object into a volume of the gel, and removing the threedimensional object after removing the first and second solvent from the gel.

9. The process of claim 1, wherein a biologically active agent is added to the porous polymeric body by at least one technique selected from the group consisting of
(i) mixing the biologically active agent with the polymer and first solvent prior to addition of the second solvent;
(ii) mixing the biologically active agent with the second solvent prior to addition to the first solvent/polymer solution ;
(iii) mixing the biologically active agent with the gel prior to removal of the first and second solvents; or
(iv) incorporating the biologically active agent within the pores of the polymeric body after removal of the first and second solvent.

10. The process of claim 1, wherein the gel is placed in contact with a separate body, after which the first and second solvent are removed, leaving the porous polymer mechanically bound to the body.

11. The process of claim 2, wherein said at least one other material is biodegradable.

12. The process of claim 2, wherein said at least one other material provides reinforcement to the porous polymer.

13. The process of claim 2, wherein said at least one other material is in the form of an article selected from the group consisting of reinforcing threads and reinforcing rings.

14. The process of claim 2, wherein said at least one other material aids in attaching the porous polymer body to host tissue.

15. The process of claim 11, wherein said at least one other material is in the form of an article selected from the group consisting of a suture and a tack.

16. The process of claim 9, wherein the biologically active agent is selected from one or more of the following: physiologically acceptable drugs, surfactants, ceramics, hydroxyapatites, tricalciumphosphates, antithrombogenic agents, antibiotics, biologic modifiers, glycosaminoglycans, proteins, hormones, antigens, viruses, cells or cellular components.

17. The process of claim 1, wherein the polymer comprises polyurethane, thereby resulting in a shaped porous polymeric body comprising polyurethane.

18. The process of claim 1, wherein the first solvent is one that is capable of substantially completely dissolving a solid form of the polymer, and further wherein the second solvent is one that does not substantially dissolve the polymer in solid form, but instead merely swells the solid polymer.

19. Use of a porous polymeric body of desired shape obtained by a process according to any of claims 1 to 18 for the manufacture of medical devices and biological prosthesis.

20. Use according to claim 19, wherein the biological prosthesis is a prosthesis for use in vascular reconstructive surgery of mammals.

21. Use according to claim 20, wherein said prosthesis for use in vascular reconstructive surgery is a vascular graft selected from peripheral grafts, arterial-to-venous access grafts or endovascular grafts.

22. Use according to claim 19, wherein the biological prosthesis is used as a substitute or repair device for the ureter, bileduct, esophagus, trachea, bladder, intestine and other hollow tissues and organs of the body, or functions as tissue conduit.

## Patentansprüche

1. Verfahren zur Erzeugung eines porösen polymeren Körpers einer gewünschten Form, das die Schritte umfasst:
a. Auswählen eines Polymers;
b. Auflösen des Polymers in einem geeigneten ersten Lösemittel unter Bildung einer Lösung;
c. Zugeben einer Menge eines geeigneten zweiten Lösemittels zu der Lösung, wobei das Gesamtvolumen der Lösung zu einem Gel einzudicken beginnt;
d. Fortsetzen der Zugabe des zweiten Lösemittels, bis die Viskosität des Gels bis zu einem Punkt zunimmt, an dem das Gel für eine Formgebung geeignet ist;
e. Formen des Gels zu einer Form; und
f. Entfernen des ersten und des zweiten Lösemittels aus dem Gel, wodurch ein Netzwerk aus kommunizierenden Poren zurückbleibt.

2. Verfahren nach Anspruch 1, das außerdem die Anordnung des Gels im Kontakt mit wenigstens einem anderen Material umfasst, so dass beim Entfernen des ersten und des zweiten Lösemittels das poröse Polymer und das wenigstens eine andere Material mechanisch aneinander gebunden sind.

3. Verfahren nach Anspruch 1, wobei das Polymer wenigstens ein Polymer umfasst, das aus der Gruppe ausgewählt ist, die besteht aus Polyurethanen, Polyharnstoffen, Polyethylenen, Polyestern und Fluorpolymeren.

4. Verfahren nach Anspruch 1, wobei das genannte erste Lösemittel ein organisches Lösemittel umfasst, das aus der Gruppe ausgewählt ist, die besteht aus N,N-Di-methylacetimid, 1-Methyl-2-pyrrolidon und Tetrahydrofuran.

5. Verfahren nach Anspruch 1, wobei das zweite Lösemittel ein organisches Lösemittel umfasst, das aus der Gruppe ausgewählt ist, die besteht aus Aceton, Chloroform, p-Dioxan, Methylenchlorid, Dimethylsulfoxid, Toluol, m-Xylol, o-Xylol und Methylethylketon.

6. Verfahren nach Anspruch 1, wobei das erste Lösemittel ein organisches Lösemittel umfasst, das aus der Gruppe ausgewählt ist, die aus der Solvent Group 3 besteht.

7. Verfahren nach Anspruch 1, wobei das zweite Lösemittel wenigstens ein organisches Lösemittel umfasst, das aus der Gruppe ausgewählt ist, die aus Solvent Group 6 oder Solvent Group 7 besteht.

8. Verfahren nach Anspruch 1, wobei die Formung des Gels wenigstens eine Technik umfasst, die aus der Gruppe ausgewählt ist, die besteht aus
(i) Verteilen des Gels auf einer offenen glatten oder texturierten Oberfläche;
(ii) Einspritzen des Gels in eine Form;
(iii) Verteilen oder Spritzen des Gels über einen dreidimensionalen Gegenstand, und Entfernen des dreidimensionalen Gegenstands nach Entfernung des ersten und des zweiten Lösemittels aus dem Gel; oder
(iv) Eindrücken eines dreidimensionalen Gegenstands in ein Volumen des Gels, und Entfernen des dreidimensionalen Gegenstands nach dem Entfernen des ersten und zweiten Lösemittels aus dem Gel.

9. Verfahren nach dem Anspruch 1, wobei dem porösen polymeren Körper ein biologisch aktives Mittel nach wenigstens einer Technik zugegeben wird, die aus der Gruppe ausgewählt ist, die besteht aus
(i) Vermischen des biologisch aktiven Mittels mit dem Polymer und dem ersten Lösemittel vor der Zugabe des zweiten Lösemittels;
(ii) Vermischen des biologisch aktiven Mittels mit dem zweiten Lösemittel vor der Zugabe zu der Lösung aus erstem Lösemittel/Polymer;
(iii) Vermischen des biologisch aktiven Mittels mit dem Gel vor der Entfernung des ersten und zweiten Lösemittels; oder
(iv) Einarbeitung des biologisch aktiven Mittels in die Poren des polymeren Körpers nach der Entfernung des ersten und des zweiten Lösemittels.

10. Verfahren nach Anspruch 1, wobei das Gel mit einem separaten Körper in Kontakt gebracht wird, wonach das erste und das zweite Lösemittel entfernt werden, wobei das poröse Polymer mechanisch an den Körper gebunden zurückbleibt.

11. Verfahren nach Anspruch 2, wobei das wenigstens eine andere Material biologisch abbaubar ist.

12. Verfahren nach Anspruch 2, wobei das wenigstens eine andere Material für das poröse Polymer eine Armierung darstellt.

13. Verfahren nach Anspruch 2, wobei das wenigstens eine andere Material in Form eines Gegenstands vorliegt, der aus der Gruppe ausgewählt ist, die aus Armierungsfäden und Verstärkungsringen besteht.

14. Verfahren nach Anspruch 2, wobei das wenigstens eine andere Material die Anfügung des porösen polymeren Körpers an ein Wirtsgewebe unterstützt.

15. Verfahren nach Anspruch 11, wobei das wenigstens eine andere Material in Form eines Gegenstands vorliegt, der aus der Gruppe ausgewählt ist, die aus einer Naht und einer Heftung besteht.

16. Verfahren nach Anspruch 9, wobei das biologisch aktive Mittel ausgewählt ist aus einem oder mehreren der folgenden: physiologisch annehmbaren Arzneimitteln, Tensiden, Keramiken, Hydroxyapatiten, Tricalciumphosphaten, antithrombogenen Mitteln, Antibiotika, biologischen Modifikatoren, Glycosaminoglycanen, Proteinen, Hormonen, Antigenen, Viren, Zellen oder Zellkomponenten.

17. Verfahren nach Anspruch 1, wobei das Polymer Polyurethan umfasst, wodurch ein geformter poröser polymerer Körper erhalten wird, der Polyurethan umfasst.

18. Verfahren nach Anspruch 1, wobei das erste Lösemittel eines ist, das in der Lage ist, eine feste Form des Polymers im Wesentlichen vollständig aufzulösen, und wobei außerdem das zweite Lösemittel eines ist, das das Polymer in fester Form nicht wesentlich auflösen kann, sondern stattdessen das feste Polymer nur aufquellen läßt.

19. Verwendung eines porösen polymeren Körpers einer gewünschten Form, der nach einem Verfahren nach irgendeinem der Ansprüche 1 bis 18 erhalten wird, zur Herstellung von medizinischen Vorrichtungen und biologischen Prothesen.

20. Verwendung nach Anspruch 19, wobei die biologische Prothese eine Prothese zur Verwendung bei der Gefäßrekonstruktions-Chirurgie von Säugetieren ist.

21. Verwendung nach Anspruch 20, wobei die genannte Prothese zur Verwendung bei der Gefäßrekonstruktions-Chirurgie ein Gefäßimplantat ist, das ausgewählt ist aus peripheren Implantaten, Implantaten zur Arterien-Venen-Verbindung oder endovaskulären Implantaten.

22. Verwendung nach Anspruch 19, wobei die biologische Prothese verwendet wird als eine Ersatz- oder Reparatur-Vorrichtung für den Harnleiter, den Gallenleiter, den Ösophagus, die Luftröhre, die Blase, den Darm und andere hohle Gewebe und Organe des Körpers, oder als Gewebeleitung funktioniert.

## Revendications

1. Procédé pour créer un corps polymère poreux de forme souhaitée, comprenant les étapes de :
a. sélection d'un polymère ;
b. dissolution du polymère dans une premier solvant approprié pour former une solution;
c. addition d'une quantité d'un deuxième solvant approprié à la solution, ce par quoi l'entièreté de la solution commence à s'épaissir en un gel ;
d. poursuite de l'addition du deuxième solvant jusqu'à ce que la viscosité du gel augmente jusqu'à un point où le gel est approprié pour la mise en forme ;
e. mise en forme du gel ; et
f. élimination des premier et deuxième solvants du gel, pour laisser un réseau poreux en inter-communication.

2. Procédé selon la revendication 1, comprenant en outre le placement du gel au contact d'au moins un autre matériau, ce par quoi lors de l'élimination du premier et du deuxième solvant, le polymère poreux et au moins un autre matériau sont liés mécaniquement l'un à l'autre.

3. Procédé selon la revendication 1, dans lequel le polymère comprend au moins un polymère choisi parmi le groupe consistant en les polyuréthannes, les polyurées, les polyéthylènes, les polyesters et les fluoropolymères.

4. Procédé selon la revendication 1, dans lequel ledit premier solvant comprend un solvant organique choisi parmi le groupe consistant en le N,N-diméthylacétimide, la 1-méthyl-2-pyrrolidone et le tétrahydrofuranne.

5. Procédé selon la revendication 1, dans lequel ledit deuxième solvant comprend un solvant organique choisi parmi le groupe consistant en l'acétone, le chloroforme, le p-dioxanne, le chlorure de méthylène, le diméthylsulfoxyde, le toluène, le m-xylène, l'o-xylène et la méthyléthylcétone.

6. Procédé selon la revendication 1, dans lequel ledit premier solvant comprend un solvant organique choisi parmi le groupe consistant en un solvant du groupe 3.

7. Procédé selon la revendication 1, dans lequel ledit deuxième solvant comprend au moins un solvant organique choisi parmi le groupe consistant en un solvant du groupe 6 ou un solvant du groupe 7.

8. Procédé selon la revendication 1, dans lequel la mise en forme du gel comprend au moins une technique choisie parmi le groupe consistant en :
(i) l'étalement du gel sur une surface lisse ou texturée ouverte ;
(ii) l'injection du gel dans un moule;
(iii) l'étalement ou l'injection du gel sur un objet tridimensionnel, et l'élimination de l'objet tridimensionnel après élimination du premier et du deuxième solvant depuis le gel, ou
(iv) l'introduction forcée d'un objet tridimensionnel dans un volume du gel, et l'élimination de l'objet tridimensionnel après élimination du premier et du deuxième solvant depuis le gel.

9. Procédé selon la revendication 1, dans lequel un agent biologiquement actif est ajouté au corps polymère poreux par au moins une technique choisie parmi le groupe consistant en :
(i) le mélange de l'agent biologiquement actif au polymère et au premier solvant, avant l'addition du deuxième solvant ;
(ii) le mélange de l'agent biologiquement actif au deuxième solvant, avant l'addition de la solution premier solvant/polymère ;
(iii) le mélange de l'agent biologiquement actif au gel avant élimination du premier et du deuxième solvant ; ou
(iv) l'incorporation de l'agent biologiquement actif dans les pores du corps polymères après élimination du premier et du deuxième solvant.

10. Procédé selon la revendication 1, dans lequel le gel est mis en contact avec un corps séparé, après quoi le premier et le deuxième solvant sont séparés, laissant le polymère poreux lié de manière mécanique au corps.

11. Procédé selon la revendication 2, dans lequel ledit au moins un autre matériau est biodégradable.

12. Procédé selon la revendication 2, dans lequel ledit au moins un autre matériau procure un renforcement au polymère poreux.

13. Procédé selon la revendication 2, dans lequel ledit au moins un autre matériau a la forme d'un article choisi parmi le groupe consistant en les vis de renforcement et les anneaux de renforcement.

14. Procédé selon la revendication 2, dans lequel ledit au moins un autre matériau aide à attacher le corps polymère poreux aux tissus mous.

15. Procédé selon la revendication 11, dans lequel ledit au moins un autre matériau a la forme d'un article choisi parmi le groupe consistant en une suture et une agrafe.

16. Procédé selon la revendication 9, dans lequel l'agent biologiquement actif est choisi parmi un ou plusieurs des suivants : médicaments physiologiquement acceptables, tensioactifs, céramiques, hydroxyapatites, phosphates tricalciques, agents antithrombogènes, antibiotiques, modifiants biologiques, glycosamino-glycanes, protéines, hormones, antigènes, virus, cellules ou composants cellulaires.

17. Procédé selon la revendication 1, dans lequel le polymère comprend un polyuréthanne, ce qui résulte en un corps polymère poreux formé comprenant un polyuréthanne.

18. Procédé selon la revendication 1, dans lequel le premier solvant est celui qui est capable de sensiblement complètement dissoudre une forme solide du polymère, et en outre, dans lequel le deuxième solvant est celui qui ne dissous sensiblement pas le polymère sous forme solide, mais qui gonfle simplement le polymère solide.

19. Utilisation d'un corps polymère poreux de forme désirée, obtenu par un procédé selon l'une quelconque des revendications 1 à 18, pour la préparation de dispositifs médicaux et prothèses biologiques.

20. Utilisation selon la revendication 19, dans laquelle la prothèse biologique est une prothèse à utiliser dans la chirurgie de reconstruction vasculaire des mammifères.

21. Utilisation selon la revendication 20, dans laquelle ladite prothèse à utiliser dans la chirurgie de reconstruction vasculaire est une greffe vasculaire choisie parmi les greffes périphériques, les greffes d'accès artériel-à-veineux et les greffes endovasculaires.

22. Utilisation selon la revendication 19, dans laquelle la prothèse biologique est utilisée comme substitut ou dispositif de réparation de l'urètre, du canal cholédoque, de l'oesophage, de la trachée, de la vessie, de l'intestin ou d'autres tissus creux et organes du corps, ou fonctionne comme un conduit tissulaire.
